Europäisches Patentamt

European Patent Office

Office européen des brevets

(19)

(11) Veröffentlichungsnummer : **0 443 356 B1**

(12) **EUROPÄISCHE PATENTSCHRIFT**

(45) Veröffentlichungstag der Patentschrift :
28.07.93 Patentblatt 93/30

(51) Int. Cl.$^5$ : **A61K 7/09**

(21) Anmeldenummer : **91101391.0**

(22) Anmeldetag : **02.02.91**

(54) Verfahren zur verformenden Behandlung von Keratinfasern sowie Mittel zur Durchführung des Verfahrens.

(30) Priorität : **03.02.90 DE 4003217**

(43) Veröffentlichungstag der Anmeldung :
**28.08.91 Patentblatt 91/35**

(45) Bekanntmachung des Hinweises auf die
Patenterteilung :
**28.07.93 Patentblatt 93/30**

(84) Benannte Vertragsstaaten :
**DE FR GB**

(56) Entgegenhaltungen :
**EP-A- 0 256 462**
**EP-A- 0 257 256**
**GB-A- 2 153 865**
**US-A- 2 540 494**

(73) Patentinhaber : **HANS SCHWARZKOPF GmbH**
**Hohenzollernring 127/129**
**W-2000 Hamburg 50 (DE)**

(72) Erfinder : **Höcker, Hartwig, Prof.**
**Dr.rer.nat.Chem.**
**Am Dorbach 23**
**W-5100 Aachen (DE)**
Erfinder : **Blankenburg, Günter, Prof. Dr.-Ing.**
**Gierlichstrasse 50**
**W-5120 Herzogenrath (DE)**
Erfinder : **Nöthen, Johannes, Dipl.-Ing. Chem.**
**Saarstrasse 80**
**W-5100 Aachen (DE)**
Erfinder : **Tolkiehn, Klaus, Dr. rer. nat. Chem.**
**Sülldorfer Brooksweg 86**
**W-2000 Hamburg 56 (DE)**
Erfinder : **Hohm, Gerhard, Dipl.-Ing. FH**
**Erlenstrasse 12**
**W-2081 Heist (DE)**

EP 0 443 356 B1

Jouve, 18, rue Saint-Denis, 75001 PARIS

## Beschreibung

Die Erfindung betrifft ein Verfahren zur verformenden Behandlung von Keratinfasern, bei dem die Keratinfasern der Einwirkung einer reaktiven reduzierenden Lösung ausgesetzt werden, die Reduktionsmittel zum Aufbrechen der in den Fasern vorhandenen Cystin-Bindungen enthält, nach der Einwirkung dieser Lösung gespült wird, die Fasern der Einwirkung eines Oxidationsmittels ausgesetzt werden, um Cystin-Bindungen wieder zurückzubilden, und gegebenenfalls nochmals gespült wird.

Unter keratinhaltigen natürlichen Fasern werden gemäß der Erfindung, wie gemäß dem Stand der Technik, Menschenhaar, Tierhaar, insbesondere Wolle von verschiedenen Tieren wie Schafwolle, Kamelhaar, Alpaka, Mohair, Kaschmir, Karakulwollem Jakhaar verstanden. Ein besonders wichtiges Anwendungsgebiet ist das lebende Menschenhaar, wenn es dem Dauerwellprozeß unterworfen wird. Das Verfahren gemäß der Erfindung kann aber in gleicher Weise bei der industriellen Bearbeitung von keratinhaltigen natürlichen Fasern angewandt werden, die einer verformenden Behandlung unterworfen werden sollen. Beispiele hierfür sind die Flächenfixierung von wollhaltiger Flächenware, die Garnfixierung, die Faltenfixierung bei der Bekleidungsherstellung, wie Dauerbügelfalte, Plissee, usw..

Verfahren der eingangs beschriebenen Art sind insbesondere zur Erzeugung von Dauerwellen des lebenden Haares seit langem bekannt (US-A-2,261,094) und werden auch heute in größtem Umfang angewandt. Dieses Verfahren wird nachfolgend "Dauerwellprozeß" bezeichnet, obwohl es, wie erwähnt, nicht nur auf das lebende Haar angewandt werden kann. Die Fasern werden dazu zweckmäßig zunächst mit Wasser genetzt. Dann erfolgt eine Reduktion der Disulfidbrücken durch die Einwirkung der Reduktionsmittel, die zu einer Reduktion der Disulfidbrücken (Cystin-Bindungen) führt. Danach wird nach dem Stand der Technik mit Wasser gespült. Anschließend erfolgt die Reoxidation, wobei die Fasern in der gewünschten Verformung, z.B. auf Lockenwicklern, gehalten werden. Zweckmäßig wird danach das Oxidationsmittel, vorzugsweise mit Wasser, ausgespült. Es sind viele Modifikationen dieses Grundverfahrens bekannt. Diese Modifikationen umfassen bestimmte Zusammensetzungen der Reduktionslösungen und/oder Oxidationslösungen sowie gegebenenfalls zusätzliche Schritte, z.B. Behandlung der Fasern mit einer Proteinerweichungslösung (DE-A-35 03 762, Priorität USA Ser.No. 577,169 und 577,180; DE-C-1 034 820; US-A-2,437,965 und 2,577,710); allen diesen Verfahren ist aber gemeinsam, daß zwischen der Einwirkung des Reduktionsmittels und der Einwirkung des Oxidationsmittels gespült wird, und zwar nach dem Stand der Technik ausschließlich mit Wasser.

All diesen Verfahren ist aber gemeinsam, daß Schädigungen der Fasern eintreten, die zu Trockenheit, Sprödigkeit, leichterem Bruch, vermindertem Glanz und erschwerter Kämmbarkeit führen können

In EP-A-0 257 256 ist im Anspruch 1 ein Verfahren zum Wellen und Glätten von Humanhaar beschrieben, bei dem das Haar in aufgerollter oder geglätteter Form vorliegt und im Kontakt mit einer ersten wäßrigen Lösung bei pH 5,5 bis 8,5; die darin gelöstes wasserlösliches Mercaptan in einer Konzentration von 0,1 bis 1,5 molar zusammen mit einem wasserlöslichen Sulfit, Bisulfit oder Hydrosulfit mit einer Konzentration von 0,1 bis 1,5 molar enthalten, gehalten wird, um dem Haar eine gewellte oder geglättete Form zu verleihen, wobei das molare Verhältnis des genannten Sulfits, Bisulfits und/oder Hydrosulfits zu Mercaptan zwischen 5:1 bis 1:7,5 beträgt und den Zustand des gewellten oder geglätteten Haares wiederherstellt und hierzu dieses mit einer wässrigen Lösung bei pH 7 bis 12 behandelt wird, die frei von vernetzenden, oxidierenden oder reduzierenden Mitteln ist. In der Beschreibung wird auf Seite 3, Absatz 2 in den Zeilen 15 bis 16 angegeben, daß bei dem Wiederherstellen Wasser allein verwendet werden kann. Im Beispiel 1 wird auf Seite 6 in Zeile 34 angegeben, daß die Wellösung durch Spülen mit Leitungswasser in 2 Minuten entfernt wird. Erst danach wurde das handtuchtrockene Haar mit der dort angegebenen Wiederherstellungslösung (pH 10) im aufgerollten Zustand gesättigt. In den Beispielen 2 bis 6 wird ebenfalls, wie im Beispiel 1 angegeben, stets mit Leitungswasser gespült. In den Beispielen 5 und 6 wird nach dieser Wasserspülung wiederum eine Wiederherstellungslösung aufgetragen.

Durch das Spülen mit Leitungswasser nach dem Reduktionsschritt müssen, wie schon vorstehend erläutert, Schädigungen der Keratinfasern eintreten. EP-A-0 256 462 beschreibt im Anspruch 1 ein Verfahren zur dauerhaften Haarverformung, bei welchem man das Haar vor und/oder nach einer mechanischen Verformung mit der wäßrigen Zubereitung einer keratinreduzierenden Substanz behandelt, das so behandelte Haar nach einer Einwirkungszeit mit Wasser spült und durch Behandlung mit einer wäßrigen Zubereitung eines Oxidationsmittels fixiert, und das so behandelte Haar nach einer Einwirkungszeit mit Wasser spült, dadurch gekennzeichnet, daß man im Anschluß an die oxidative Fixierung das noch mechanisch verformte Haar mit einer wäßrigen Zubereitung nachbehandelt, die ein gelöstes Aluminium(III)-Salz und eine Hydroxydi- oder Tricarbonsäure mit 4 bis 5 C-Atomen enthält. Gemäß den Angaben im Anspruch 1 und in den Beispielen werden stets die bereits fixierten Haare mit wäßrigen Zubereitungen (Dauerwell-Haarfestiger, Dauerwell-Konditionierungsmittel, Dauerwell-Haarwasser oder Dauerwell-Spülung) nachbehandelt, die ein gelöstes Aluminium (III)-Salz und eine Hydroxydi- oder Tricarbonsäure mit 4 bis 6 C-Atomen enthält. Auch bei diesem Verfahren wird das Haar nach der Behandlung mit einer wäßrigen Zubereitung einer keratinreduzierenden Substanz mit Wasser gespült, so daß bei diesem Verfahrensschritt die schon vorstehend erläuterten Schädigungen der Keratinfasern auftreten müssen.

GB-A-2 153 865 betrifft ein Verfahren zum dauerhaften Umstrukturieren von Haar, bei dem das Haar der Einwirkung einer reaktiven reduzierenden Lösung ausgesetzt wird, die wenigstens ein Reduktionsmittel zum Aufbrechen der Cystin-Bindung enthält und ein Oxidationsmittel auf das Haar aufgetragen wird, um die Cystin-Bindung wieder zurückzubilden, dadurch gekennzeichnet, daß

a) das Haar über einen Zeitraum mit der reaktiven Reduktionslösung in Kontakt gebracht wird, die ausreicht, um ein im wesentlichen maximales Aufbrechen der Cystin-Bindung zu erreichen;

b) das Haar abgestreift wird, um die reaktive Reduktionslösung zu entfernen;

c) das Haar über einen Zeitraum mit einer Protein-Erweichungslösung in Kontakt gebracht wird, die ausreicht, um die Protein-Erweichung im Haar zu bewirken und die gewünschte umstrukturierte Konfiguration zu erhalten, danach ein Oxidationsmittel angewandt wird, um die Cystin-Bindungen wieder zurückzubilden und die umstrukturierte Konfiguration zu fixieren, wobei die Protein-Erweichungslösung eine wässrige Lösung mit einem pH-Wert von etwa 2 bis etwa 10 ist, die eine wässrige Lösung von mindestens einem Protein-Erweichungsmittel umfaßt, ausgewählt aus der Gruppe bestehend aus einem mehrwertigen Ion, einer wasserlöslichen hydroxyorganischen Verbindung, enthaltend bis zu 4 Kohlenstoffatomen und mindestens eine Hydroxylgruppe sowie deren Mischungen. Bei diesem Verfahren verbleibt nach der Behandlung mit einer wäßrigen Zubereitung einer keratinreduzierenden Substanz ein gewisser Anteil auf den Keratinfasern, da diese nicht gespült werden, sondern die Zubereitung abgestreift wird, so daß die nicht abgestreiften Anteile schädigend auf die Keratinfasern einwirken. Danach wird das abgestreifte Haar etwa 10 Minuten mit der dort beschriebenen Protein-Einweichungslösung in Kontakt gebracht, so daß die absolute Menge an keratinreduzierender Substanz, jedoch in verdünnter Form, immer noch schädigend vorliegt.

US-A-2 540 494 umfaßt in Anspruch 1 eine Kombination in Zubereitungen für das permanente Kaltwellen von Haar auf Humanköpfen mit einer ersten wäßrigen Lösung, enthaltend ein alkalisches Reagenz zum Quellen des Haares, ein haarwellendes Mercaptan zum Aufbrechen der Disulfidbindungen des Haarkeratins und zum Durchdringen des gequollenen Haares und dessen aufgebrochenen Keratin und ein Protein, welches in alkalischer Lösung löslich ist und in einer sauren Lösung ausfällt, wobei das genannte Protein in der genannten ersten Lösung aufgelöst vorliegt, um das gequollene Haar und das aufgebrochene Kreatin zu durchdringen; und eine zweite danch zu verwendende wässerige Lösung, enthaltend ein Oxidationsmittel, um substantiell die Disulfidbindungen des Haarkeratins wiederherzustellen und ein Säuerungsmittel (ein anderes als das Oxidationsmittel), um der zweiten Lösung einen pH unter dem isoelektrischen Punkt des Proteins zu verleihen, um das eingedrungene Protein auszufällen, welches gleichzeitig wegen der Anwesenheit des eingedrungenen organischen Mercaptans gehärtet wird. Dort ist in Spalte 8 Zeilen 1 bis 4 angegeben, daß die üblichen Wasserspülungen nach dem Behandeln des Haares mit Well- bzw. Fixierlösungen ausgeführt werden, wie dies in die Zeichnung im Arbeitsablauf etwa in der Mitte mit der Bezeichnung rinse Water verdeutlicht worden ist. Ferner sind dort in Spalte 2, Zeile 3 bis Zeile 43 Beispiele für wasserlösliche alkalische Mittel angegeben, die der Mercaptanlösung zugegeben werden können. Durch das Spülen mit Wasser nach der Behandlung mit der keratinreduzierenden Lösung müssen die bereits vorstehend erläuterten Schädigungen der Keratinfasern eintreten.

Der Erfindung liegt die Aufgabenstellung zugrunde, ein Verfahren zu finden, das solche Schädigungen der Fasern vermindert. Die Erfinder haben in diesem Zusammenhang erstmals festgestellt, daß beim Spülen der Fasern mit Wasser nach der Einwirkung des Reduktionsmittels eine kurzzeitige Quellung der Fasern eintritt, die dadurch bewirkt wird, daß das Wasser in die Fasern eindringt, in denen noch Reduktionsmittellösung enthalten ist. Die Erfinder haben weiterhin festgestellt, daß dennoch am Ende des Spülprozesses mit Wasser der Durchmesser der Fasern nicht wesentlich größer als am Ende des Reduktionsschrittes, sondern meist sogar geringer ist. Weiterhin haben die Erfinder festgestellt, daß die Schädigung der Fasern auf diese selbst sehr kurzzeitige Durchmesserzunahme der Fassern zurückzuführen ist, und daß diese Schädigung im wesentlichen irreversibel ist. Die Erfinder haben weiterhin festgestellt, daß dann, wenn eine selbst kurzzeitige Durchmesserzunahme während des Spülens verhindert wird, die Schädigung der Fasern sehr viel geringer ist. Gegenstand der Erfindung ist demgemäß das Verfahren der eingangs beschriebenen Gattung, das dadurch gekennzeichnet ist, daß nach dem Einwirken der reduzierenden Lösung mit einer solchen wässrigen Lösung von gegenüber den Fasern inerten Stoffen gespült wird, die bewirkt, daß zu keinem Zeitpunkt während des Spülvorganges der Durchmesser der Fasern um mehr als 30% größer ist als am Ende des Reduktionsschrittes

Man hat bisher geglaubt, daß die Schädigung der Keratinfasern, insbesondere der Haare während des Dauerwellprozesses, allein durch das Reduktionsmittel in der ersten Stufe der Dauerwellbehandlung bewirkt wird. Es wurde nun überraschenderweise gefunden, daß eine Schädigung selbst bei Anwendung verschärfter Bedingungen (höherer pH-Wert und/oder höhere Konzentration des Reduktionsmittels während des Reduktionsschrittes) eine Schädigung des Haares verhindert oder vermindert werden kann, wenn zum Spülen nicht Wasser, sondern die Flüssigkeiten gemäß der Erfindung verwendet werden.

Bei den Entgegenhaltungen mit den dortigen Behandlungslösungen, wird nur tropfenweise, also insgesamt sehr wenig Behandlungslösung auf das Haar aufgetragen, so daß auch nur wenig Wirkstoff zur Anwendung kommt.

Da bei der Erfindung nicht mit geringen Mengen "behandelt wird"sondern ein echtes Spülen stattfindet (laut Beschreibung 5 min.) kommen bei der vorliegenden Erfindung wirklich große Mengen an Flüssigkeit und an Wirkstoff zur Anwendung.

Besonders bedeutsam ist, daß beim Spülen mit Wasser nach dem Stand der Technik Quellungen, d.h. Durchmesservergrößerungen der Fasern (Haare) eintreten, die zum größten Teil irreversibel sind. Es ist das Verdienst der Erfinder der vorliegenden Erfindung gefunden zu haben, daß eine solche irreversible Schädigung weitgehend vermieden werden kann, wenn zum Spülen nicht Wasser, sondern die Flüssigkeiten gemäß der Erfindung eingesetzt werden.

Vor der Anwendung des Reduktionsmittels werden zweckmäßig die Keratinfasern, wie gemäß dem Stand der Technik, vorzugsweise mit Wasser, benetzt. Als Reduktionsmittel können die üblichen auf diesem technischen Gebiet bekannten Lösungen verwendet werden, es wird dazu u.a. auf die oben genannten Patentschriften verwiesen. Besonders bei der verformenden Behandlung von lebendem Haar, d.h. im Dauerwellverfahren, wird als Reduktionsmittel Thioglykolsäure verwendet. Der pH-Wert der Reduktionslösung kann von saurem pH-Wert von etwa 5 bis zum alkalischen pH-Wert von etwa 10 schwanken. Besonders bevorzugte pH-Werte sind - wie gemäß dem Stand der Technik - solche im Bereich von etwa 6 bis etwa 10 und besonders von etwa 6 bis etwa 9,5. Die Behandlung mit dem Reduktionsmittel erfolgt während etwa 5 bis etwa 30 Minuten. Der Überschuß der reduzierenden Lösung wird, wie nach dem Stand der Technik, zweckmäßig von den Fasern abgestreift.

Gemäß der Erfindung wird nunmehr nicht mit Wasser, sondern mit der oben definierten wässrigen Lösung von gegenüber den Fasern inerten Stoffen gespült. Dabei ist es zweckmäßig, das eine solche Lösung verwendet wird, die bewirkt, daß zu keinem Zeitpunkt während des Spülvorganges der Durchmesser der Fasern um mehr als 20 %, besonders bevorzugt um mehr als 10 % größer ist als am Ende des Reduktionsschrittes. Ganz besonders bevorzugt ist, daß mit einer Lösung gespült wird, die bewirkt, daß zu keinem Zeitpunkt des Spülvorganges der Durchmesser der Fasern zunimmt.

Von den Erfindern durchgeführte Versuche und Aufnahmen mit dem Rasterelektronenmikroskop beweisen deutlich, daß beim Verfahren gemäß der Erfindung durch das Unterdrücken der Quellung beim Spülen mit einer Lösung gemäß der Erfindung eine Schädigung der Haaroberfläche wirksam verhindert wird. Sie weist wesentlich geringere Veränderungen auf als bei der Spülung mit Wasser. Der natürliche Griff und Glanz der ungeschädigten, gesunden Haare bzw. Fasern bleibt weitestgehend erhalten.

Wird die Energieaufnahme eines nassen Haares bei einer Dehnung von 20 % vor und nach dem Behandlungszyklus ermittelt, so zeigen Reduktionssysteme für die Spülung mit Wasser eindeutig eine geringere Energieaufnahme als bei der Spülung mit der Lösung gemäß der Erfindung. Je geringer der Quotient der Energieaufnahme des Haares bei der Dehnung nach und vor einem Behandlungszyklus ist, desto geringer sind auch die strukturellen Schäden des Haares. Wird zur Spülung nach der Reduktion eine Lösung gemäß der Erfindung verwendet, so hat das einen positiven Einfluß auf die Erhaltung des strukturellen Zustandes der Keratinfaser.

Das Ergebnis einer dauerhaften Verformung von Keratinfasern wird durch Einsatz einer Spüllösung gemäß der Erfindung besser vorhersehbar als bei Verwendung von Wasser. Die hohe unkontrollierte Quellung des Haares beim Spülen mit Wasser führt zu sehr unterschiedlichen Verformungsgraden, während das Spülen mit einer Lösung gemäß der vorliegenden Erfindung zu wesentlich gleichmäßigeren Verformungsergebnissen führt.

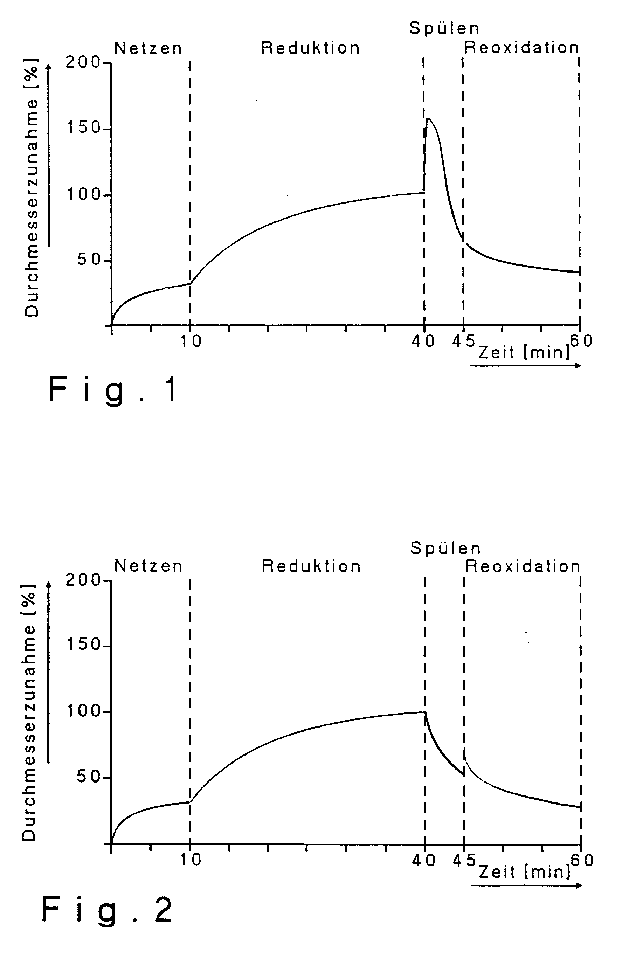

Die Untersuchungen der Erfinder haben ergeben, daß während des Spülschrittes mit Wasser für eine kurze Zeitspanne eine deutliche Erhöhung des Haardurchmessers stattfindet, wie Figur 1 zeigt. In Figur 1 ist die Durchmesserzunahme der Fasern in % gegen die Zeit in Minuten aufgetragen. Die unmittelbar nach Beginn der Spülung eintretende starke Quellung der Fasern kann sowohl zu einer starken, irreversiblen Schädigung der Faseroberfläche als auch zu einer Schwächung der inneren Faserstruktur führen.

Bei Verwendung der Spüllösung gemäß der Erfindung wird eine Quellung der Fasern beim Ausspülen des Reduktionsmittels wirksam verhindert, wie Figuren 1 und 2 zeigen. Zwar kann, wie in Figur 2 zu ersehen, zu Beginn der anschließenden Reoxidation eine geringe Durchmesserzunahme erfolgen. Am Ende der Reoxidation wird jedoch ein geringerer Durchmesser der Keratinfasern erreicht als beim Spülen mit Wasser, wie aus Figur 1 ersichtlich ist. Dieser geringere Enddurchmesser kann als Indiz dafür verwertet werden, daß die Schädigung der Keratinfaser beim Spülen mit einer Lösung gemäß der Erfindung insgesamt geringer ist.

Die Figur 2 zeigt den Quellungsverlauf für einen Dauerwellprozeß, bei dem nicht mit Wasser, sondern mit

4

einer Spülflüssigkeit gemäß der Erfindung gespült wurde.

Aufgrund des obigen von den Erfindern ermittelten Sachverhaltes kann das Verfahren gemäß der Erfindung auch so definiert werden, daß mit einer solchen wässrigen Lösung von gegenüber den Fasern inerten Stoffen gespült wird, die bewirkt, daß gegenüber dem oder im Vergleich zum Spülen mit destilliertem Wasser eine Verminderung der maximalen Zunahme des Faserdurchmessers von mindestens 20 %, vorzugsweise mindestens 50 %, bewirkt wird. Der Prozentsatz der Verminderung der maximalen Zunahme des Faserdurchmessers wird wie folgt berechnet:

DW = Maximale Zunahme des Faserdurchmessers beim Spülen mit destilliertem Wasser in Prozent.

EW = Maximale Zunahme des Faserdurchmessers beim Spülen mit der Flüssigkeit gemäß der Erfindung in Prozent

$$\frac{DW - EW}{DW} \cdot 100$$

Unter inerten Stoffen werden gemäß der Erfindung solche verstanden, die im wesentlichen keine nachteiligen Einwirkungen auf die Qualitätseigenschaften der Fasern haben. Sofern das Verfahren auf lebendes Haar angewandt wird, müssen naturgemäß solche inerten Stoffe verwendet werden, die auch auf die Kopfoberfläche keine wesentlichen nachteiligen oder unangenehmen Wirkungen haben. Es hat sich in diesem Sinne als vorteilhaft erwiesen, wässrige Natriumchlorid-Lösungen einzusetzen.

Die wässrige Natriumchloridlösung enthält zweckmäßig 10 g bis 350 g Natriumchlorid pro Liter Wasser. Die untere Grenze liegt vorteilhaft bei 75 g, die obere Grenze bei 300 g.

Die gemäß der Erfindung verwendeten Spüllösungen haben einen gegenüber destilliertem Wasser erhöhten osmotischen Druck, zweckmäßig von mindestens 4,5 x $10^5$ Pascal (Pa) bei 25 °C. Vorzugsweise haben die Spüllösungen einen osmotischen Druck im Bereich von etwa 6 x $10^5$ bis 380 x $10^5$ Pa. Die untere Grenze liegt besonders bevorzugt bei etwa 60 x $10^5$ Pa und die obere Grenze bevorzugt bei etwa 250 x $10^5$ Pa.

Beim Spülen mit Wasser gemäß dem Stand der Technik ist, wie die Erfinder festgestellt haben, die Durchmesserzunahme im allgemeinen um so größer, je höher der pH-Wert des Reduktionsmittels und je größer die Konzentration der reduzierenden Substanzen in der Reduktionslösung sind. Je höher also der pH-Wert und je größer die Konzentration des Reduktionsmittels, desto größer ist der gemäß der Erfindung erzielte Effekt einer Verminderung der Durchmesserzunahme der Fasern.

Die Höhe des osmotischen Druckes der Spüllösung kann dem pH-Wert und der Konzentration der Reduktionslösung angepaßt werden. Aus den oben dargelegten Gründen sollte der osmotische Druck der Spüllösung um so höher sein, je höher der pH-Wert und/oder je höher die Konzentration der Reduktionslösungen sind. Bei Verwendung einer Reduktionslösung mit 10 Gew.-% Thioglykolsäure, deren pH-Wert mit Ammoniak auf 9 eingestellt ist, genügt bereits eine 8 gewichtsprozentige NaCl-Spüllösung, um die Durchmesserzunahme eines kaukasischen Humanhaares während des Spülprozesses zuverlässig zu verhindern. Ist der pH-Wert der Reduktionslösung mit Natronlauge auf 9 eingestellt, wird bei sonst gleichen Randbedingungen eine wässrige Spüllösung mit 12 Gew.-% NaCl benötigt, um die Durchmesserzunahme während des Spülschrittes zu unterdrücken.

Die Spüllösung gemäß der Erfindung kann -wie die anderen angewandten Lösungen- insbesondere bei der Anwendung am lebenden Haar noch übliche Zusatzmittel enthalten wie Parfüm, Tenside, Konservierungsmittel, antibakterielle Mittel, Farbstoffe, Verdickungsmittel usw.

Nach dem Spülen werden die Fasern wie gemäß dem Stand der Technik der Einwirkung eines Oxidationsmittels ausgesetzt. Es können die aus dem Stand der Technik bekannten Oxidationsmittel eingesetzt werden. Auch hierzu wird auf die eingangs erwähnten Patentschriften verwiesen.

Das Spülmittel gemäß der Erfindung sowie das Oxidationsmittel werden zweckmäßig abgestreift, ehe der nächste Behandlungsschritt erfolgt. Nach der Einwirkung des Oxidationsmittels kann, wie gemäß dem Stand der Technik, mit Wasser gespült werden. In diesem Verfahrensschritt treten auch beim Spülen mit Wasser keine erheblichen nachteiligen Einflüsse auf die Qualität der Fasern auf.

Die Dauer des Spülvorganges ist wie gemäß dem Stand der Technik beim Spülen mit Wasser. Es kommt lediglich darauf an, daß das Reduktionsmittel genügend ausgespült wird.

Die Erfindung wird anhand der Beispiele weiter erläutert.

Beispiel 1

Es wird ein Dauerwellverfahren durchgeführt mit folgenden Verfahrensparametern:

1. 10 min Netzen
2. 30 min Reduktion
3. 5 min Spülung
4. 15 min Reoxidation

Die Einsatztemperatur aller Lösungen beträgt 21 °C.
Netzen wird mit destilliertem Wasser durchgeführt.
Rezeptur für 100 g Reduktionslösung:
12,5 g Thioglykolsäure (80 Gew.% in $H_2O$),
mit Ammoniak auf pH 9 eingestellt.
Diese Reduktionslösung wird nachfolgend als "Lösung 1" bezeichnet. Zum Spülen wurde eine Lösung von 8 g Natriumchlorid in 92 g destilliertem Wasser verwendet.
Rezeptur für 100 g Reoxidationsmittel:
2,5 g Wasserstoffperoxid
mit Phosphorsäure auf pH 2,5 eingestellt.

Vergleichsversuch 1

Es wurde wie in Beispiel 1 gearbeitet, wobei jedoch zum Spülen destilliertes Wasser verwendet wurde.
Die Ergebnisse sind in den nachfolgenden Tabellen 1 bis 3 zusammengefaßt.

Beispiel 2

Es wurde wie in Beispiel 1 gearbeitet, wobei jedoch die Reduktionslösung nicht mit Ammoniak, sondern mit Natronlauge auf pH 9 eingestellt worden war.
Diese Reduktionslösung wird nachfolgend als "Lösung 2" bezeichnet. Zum Spülen wurde eine Lösung von 12 g Natriumchlorid in 88 g destilliertem Wasser verwendet.

Vergleichsversuch 2

Es wurde wie in Beispiel 2 gearbeitet, wobei jedoch zum Spülen destilliertes Wasser verwendet wurde.
Tabelle 1 gibt eine Übersicht der Ergebnisse über die Beispiele 1 bis 2 und die Vergleichsversuche 1 und 2.

## Tabelle 1

| Beispiel 1 | Vergleichsversuch 1 | Beispiel 2 | Vergleichsversuch 2 |
|---|---|---|---|
| Netzen | Netzen | Netzen | Netzen |
| Lösung 1 | Lösung 1 | Lösung 2 | Lösung 2 |
| Spülung mit Na Cl-Lösung | Spülung mit destilliertem Wasser | Spülung mit NaCl-Lösung | Spülung mit destilliertem Wasser |
| Reoxidation | Reoxidation | Reoxidation | Reoxidation |

**Tabelle 2**  (Quellung in %) *)

| | Reduktion Endwert | Spülen Max-Wert | Spülen Endwert | Reoxidation Endwert |
|---|---|---|---|---|
| Beispiel 1 | 74 | 74 | 58 | 39 |
| Vergleichs-versuch 1 | 74 | 94 | 46 | 37 |
| Beispiel 2 | 65 | 65 | 44 | 38 |
| Vergleichs-versuch 2 | 65 | 167 | 133 | 93 |

*) Die Quellungswerte beziehen sich auf den Durchmesser des mit Stickstoff getrockneten Haares

Aus den obigen Werten ist ersichtlich, daß nach den Vergleichsversuchen beim Spülen mit Wasser die maximale Quellung sehr hoch ist, während bei den Beispielen gemäß der Erfindung der Quellungswert am Ende der Reduktion überhaupt nicht überschritten wird.

**Tabelle 3** $\dfrac{E_{nachher}}{E_{vorher}} \times 100$ **)

| | Mittelwert in % | Standardabweichung |
|---|---|---|
| Beispiel 1 | 18,54 | 1,78 |
| Vergleichs-versuch 1 | 16,98 | 2,21 |
| Beispiel 2 | 34,93 | 4,17 |
| Vergleichs-versuch 2 | 24,81 | 1,64 |

**) $E_{vorher}$ – Energieaufnahme des unbehandelten Haares bei 20 % Dehnung im Wasser

$E_{nachher}$ – Energieaufnahme des behandelten Haares bei 20 % Dehnung im Wasser

Die obigen Werte zeigen, daß bei den Vergleichsversuchen im Vergleich zu den jeweiligen Beispielen gemäß der Erfindung das Verhältnis der Energieaufnahme des unbehandelten Haares zur Energieaufnahme des behandelten Haares deutlich geringer ist, was ein Maß für die größere Schädigung des Haares ist.

Unterschiede in den Mittelwerten zwischen Beispiel 1 und Vergleichsversuch 1 sowie zwischen Beispiel 2 und Vergleichsversuch 2 sind mit mehr als 95 % statistisch signifikant.

Beispiel 3

Durchführung des Dauerwellverfahrens wie im Beispiel 1, jedoch als Reduktionsmittel eine handelsübliche Dauerwellösung (Stylewave X von Clynol auf Basis von Ammoniumthioglycolat), als Spülung eine Lösung von 17g $Mg SO_4 . 7 H_2O$ (MG: 246,5g/mol) in 100ml entsalztem Wasser = 1,38 val/l und als Reoxidationsmittel eine handelsübliche Fixierung (Natural Styling Stabilfix, auf Basis von 2,5%iger $H_2O_2$-Lösung). Der pH-Wert der Spüllösung lag bei 5,2.

Während die Beispiele 1 und 2 zur Bestimmung des Haarquerschnittes an Einzelhaaren durchgeführt wurden, erfolgten die Dauerwellbehandlungen der Beispiele 3-17 an Haarsträhnen von etwa 3g und 15cm Länge.

Diese Haarsträhnen aus europäischem Menschenhaar waren, wie in der Praxis üblich, über Spitzenpapier auf Wickler mit 9mm Durchmesser gewickelt. Für jedes Beispiel wurden mindestens drei Strähnen nach folgendem Verfahren präpariert und ausgewertet:

**Beschreibung der Versuchsanordnung**

Die gewickelten Haarsträhnen wurden nach folgendem Zeitschema behandelt:
1. 10 min Netzen in entsalztem Wasser
2. 15 min Einwirkung des Reduktionsmittels
3. 10 min Spülung
4. 10 min Reoxidation

Während die Schritte 1. und 2. in einer offenen Präparationsschale erfolgten, wurde der Punkt 3. in einem geschlossenen System durchgeführt.

In einem 1l Zweihalskolben wurden 250ml der Spüllösung vorgelegt. In diese Salzlösung wurden die gewickelten Haarsträhnen nach der Reduktionsmitteleinwirkung gebracht. Durch Zugabe von Leitungswasser mit der Geschwindigkeit von etwa 750ml/min wurde die Salzlösung langsam kontinuierlich verdünnt. Der Zulauf erfolgte über eine Glasfritte am Boden des Kolbens, so daß eine feine Verteilung ohne Rühren gewährleistet war. Über den zweiten Kolbenhals floß die überschüssige verdünnte Spüllösung ab. Nach 10 min Spülzeit lag der Salzgehalt der Lösung unter 0,1%.

Für den Punkt 4. wurden die Strähnen aus der Spülapparatur herausgenommen, die überschüssige Lösung mit einem weichen Zellstofftuch abgetupft und die noch auf dem Wickler befindliche Strähne mit der Fixierlösung durchtränkt. Nach 8 min wurde die Strähne vom Wickler genommen und nochmals mit frischer Fixierlösung behandelt, um eine optimale Reoxidation zu gewährleisten.

**Vergleichsversuch 3**

Es wurde wie in Beispiel 3 gearbeitet, wobei jedoch zum Spülen in dem Kolben 250ml entsalztes Wasser anstelle der Salzlösung vorgelegt wurde.

Die Ergebnisse dieser Versuche wurden von mehreren Personen (mindestens 5) friseurtechnisch beurteilt. Sie sind in Tabelle 4 zusammengefaßt:

**Beispiel 4**

Durchführung eines Dauerwellverfahrens wie in Beispiel 3, jedoch als Spülung eine Lösung von 9,8g $Na_2 SO_4$ (MG: 142,04 g/mol) in 100ml entsalztem Wasser, $\triangleq$ 1,38 val/l. Der pH-Wert der $Na_2 SO_4$-Lösung lag bei 5,6.

**Beispiel 5**

Durchführung eines Dauerwellverfahrens wie in Beispiel 3, jedoch als Spülung eine Lösung von 10,3g KCl (MG: 74,56 g/mol) in 100ml entsalztem Wasser, $\triangleq$ 1,38 val/l. Der pH-Wert der KCl-Lösung lag bei 5,5.

**Beispiel 6**

Durchführung eines Dauerwellverfahrens wie in Beispiel 3, jedoch als Spülung eine Lösung von 5,85g LiCl (MG: 42,4g/mol) in 100ml entsalztem Wasser, $\triangleq$ 1,38 val/l. Der pH-Wert der LiCl-Lösung lag bei 5,1.

**Beispiel 7**

Durchführung eines Dauerwellverfahrens wie in Beispiel 3, jedoch als Spülung eine Lösung von 14,2g NaBr (MG: 102,9 g/mol) in 100ml entsalztem Wasser, $\triangleq$ 1,38 val/l. Der pH-Wert der NaBr-Lösung lag bei 6,2.

**Beispiel 8**

Durchführung eines Dauerwellverfahrens wie in Beispiel 3, jedoch als Spülung eine Lösung von 10,64g $NH_4 CH_3 COO$ (MG: 77,08g/mol) in 100ml entsalztem Wasser, $\triangleq$ 1,38 val/l. Der pH-Wert der Ammoniumacetat-Lösung lag bei 7,3.

**Beispiel 9**

Durchführung eines Dauerwellverfahrens wie in Beispiel 3, jedoch als Spülung eine Lösung von 14,8g Mg $(CH_3COO)_2$ . 4 $H_2O$ (MG: 214,5 g/mol) in 100ml entsalztem Wasser, $\triangleq$ 1,38 val/l. Der pH-Wert der Mg $(CH_3COO)_2$-Lösung lag bei 9,2.

**Beispiel 10**

Durchführung eines Dauerwellverfahrens wie in Beispiel 3, jedoch als Spülung eine Lösung von 13,5g Aluminiumlactat (MG: 294,19g/mol) in 100ml entsalztem Wasser, $\triangleq$ 1,38 val/l. Der pH-Wert der Aluminiumlactatlösung lag bei 3,5.

**Beispiel 11**

Durchführung eines Dauerwellverfahrens wie in Beispiel 3, jedoch als Spülung eine Lösung von 14,0g $MgCl_2$ . 6 $H_2O$ (MG: 203,3g/mol) in 100ml entsalztem Wasser, $\triangleq$ 1,38 val/l. Der pH-Wert der $MgCl_2$-Lösung lag bei 4,8.

**Beispiel 12**

Durchführung eines Dauerwellverfahrens wie in Beispiel 3, jedoch als Spülung eine Lösung von 16,2g Betain (MG: 117,2 g/mol) in 100ml entsalztem Wasser, $\triangleq$ 1,38 val/l. Der pH-Wert der Betain-Lösung lag bei 5,9.

**Beispiel 13**

Durchführung eines Dauerwellverfahrens wie in Beispiel 3, jedoch als Spülung eine Lösung von 10,4g Diammoniumhydrogencitrat (MG: 226,2 g/mol) in 100ml entsalztem Wasser, $\triangleq$ 1,38 val/l. Der pH-Wert der Diammoniumhydrogencitratlösung lag bei 5,4.

**Beispiel 14**

Durchführung eines Dauerwellverfahrens wie in Beispiel 3, jedoch als Spülung ein Gemisch von 8,1g Betain und 5,32g $NH_4$ $CH_3$ COO in 100ml entsalztem Wasser, $\triangleq$ 1,38 val/l. Der pH-Wert der Lösung aus Betain und Ammoniumacetat lag bei 7,2.

**Beispiel 15**

Durchführung eines Dauerwellverfahrens wie in Beispiel 3, jedoch als Spülung eine Lösung von 6,4g Ethanol (MG: 46,07 g/mol) in 100ml entsalztem Wasser.

**Beispiel 16**

Durchführung eines Dauerwellverfahrens wie in Beispiel 3, jedoch als Spülung eine Lösung von 12,7g Glycerin (MG: 92,1 g/mol) in 100ml entsalztem Wasser.

**Beispiel 17**

Durchführung eines Dauerwellverfahrens wie in Beispiel 3, jedoch als Spülung eine Lösung von 25,1g D-Sorbit (MG: 182,2 g/mol) in 100ml entsalztem Wasser.

Eine Variante des Verfahrens der Erfindung ist dadurch gekennzeichnet, daß nach Beginn des Spülens mit der wässrigen Lösung, die die gegenüber den Fasern inerten Stoffe enthält, durch kontinuierliche oder diskontinuierliche Zugabe von Wasser die Konzentration der inerten Stoffe in der Spüllösung so lange abgesenkt wird, bis nur noch mit Wasser gespült wird.

Eine weitere Variante des Verfahrens der Erfindung ist dadurch gekennzeichnet, daß

(1) nach dem Einwirken der reduzierenden Lösung in einer ersten Behandlungsstufe

(2) die Keratinfasern in einer zweiten Behandlungsstufe mit einer solchen wässrigen Lösung von gegenüber den Keratinfasern inerten Stoffen benetzt werden, die bewirkt, daß zu keinem Zeitpunkt während des Benetzungsvorganges der Durchmesser der Keratinfasern um mehr als 30% größer ist als am Ende des Reduktionsschrittes und im günstigsten Falle zu keinem Zeitpunkt des Benetzungsvorganges der Durchmesser der Keratinfasern zunimmt und

(3) in einer dritten Behandlungsstufe die benetzten Keratinfasern mit Wasser gespült werden, wobei zu keinem Zeitpunkt des Spülvorganges der Durchmesser der Keratinfasern zunimmt.

Mittel zur Durchführung des Verfahrens der Erfindung sind in den Ansprüchen 12 bis 15 beschrieben.

## Tabelle 4

| | Beurteilung des Kämm-widerstandes der einzelnen Testpersonen | | | | | Beurteilung des Griffes, (Glätte, Rauhigkeit) durch die einzelnen Testpersonen | | | | |
|---|---|---|---|---|---|---|---|---|---|---|
| | TP 1 | TP 2 | TP 3 | TP 4 | TP 5 | TP 1 | TP 2 | TP 3 | TP 4 | TP 5 |
| Beispiel 3 gegenüber Vergleich 3 | +3 | +2 | +2 | +2 | +2 | +2 | +2 | +2 | +2 | +3 |
| Beispiel 4 gegenüber Vergleich 3 | +2 | +2 | +2 | +2 | +2 | +2 | +2 | +2 | +2 | +2 |
| Beispiel 5 gegenüber Vergleich 3 | +3 | +2 | +2 | +2 | +2 | +2 | +2 | +2 | +2 | +1 |
| Beispiel 6 gegenüber Vergleich 3 | +3 | +3 | +3 | +3 | +2 | +3 | +3 | +2 | +2 | +2 |
| Beispiel 7 gegenüber Vergleich 3 | -1 | 0 | -1 | -1 | 0 | 0 | 0 | 0 | +1 | +1 |
| Beispiel 8 gegenüber Vergleich 3 | +2 | +2 | +3 | +3 | +2 | +2 | +1 | +1 | +2 | +2 |
| Beispiel 9 gegenüber Vergleich 3 | +2 | +3 | +2 | +2 | +2 | +2 | +2 | +3 | +3 | +2 |
| Beispiel 10 gegenüber Vergleich 3 | +1 | 0 | +1 | +1 | 0 | +1 | +1 | +1 | +1 | 0 |
| Beispiel 11 gegenüber Vergleich 3 | +1 | 0 | 0 | +1 | 0 | +1 | +1 | 0 | +1 | +1 |
| Beispiel 12 gegenüber Vergleich 3 | +3 | +3 | +4 | +3 | +3 | +4 | +3 | +3 | +3 | +4 |
| Beispiel 13 gegenüber Vergleich 3 | +2 | +3 | +2 | +2 | +2 | +2 | +2 | +2 | +2 | +1 |
| Beispiel 14 gegenüber Vergleich 3 | +3 | +4 | +4 | +3 | +3 | +4 | +4 | +4 | +3 | +3 |
| Beispiel 15 gegenüber Vergleich 3 | +2 | +2 | +2 | +2 | +2 | +2 | +3 | +2 | +2 | +2 |
| Beispiel 16 gegenüber Vergleich 3 | +3 | +3 | +2 | +3 | +2 | +2 | +2 | +1 | +2 | +2 |
| Beispiel 17 gegenüber Vergleich 3 | +1 | 0 | +1 | +1 | +1 | +2 | +1 | +2 | +2 | +1 |

## Zeichenerklärungen

Die Bewertung der Beispiele (3 bis 17) gegenüber jeweils dem Vergleichsversuch 3 erfolgte nach einem Notenschlüssel, in dem die einzelnen Noten folgende Bedeutung haben:

1   im direkten Vergleich noch erkennbar
2   im direkten Vergleich erkennbar
3   im direkten Vergleich gut erkennbar
4   im direkten Vergleich sehr deutlich erkennbar
+   besser als Vergleichsversuch 3
-   schlechter als Vergleichsversuch 3
0   gleichwertig

**Bewertungsparameter**

Die Bewertungen erfolgten insgesamt an feuchten Strähnen. Es wurden die Kämmfähigkeit und der Griff von jeweils zwei hintereinander aufgehängten Strähnen beurteilt, wobei die Kämmfähigkeitsprüfung erst nach der Entwirrung der Doppelsträhnen erfolgte. Die verwendeten Kämme waren Produkte der Firma Hercules und trugen die Bezeichnung Sägemann (R) 480. Die Beurteilung selbst erfolgte subjektiv durch fünf Friseurmeister.

Orientierende Untersuchungen mit weiteren Dauerwellösungen nach dem Stand der Technik auf Basis von Mercapto-Verbindungen und der Sulfiten wie diese in der einschlägigen Literatur (siehe nachfolgende Seite) beschrieben sind, haben unter Verwendung des erfindungsgemäßen Spülverfahrens ergeben, daß die erfindungsgemäßen Effekte sicher erreicht werden.

**Literaturangaben**

C.R. Robbins
Chemical and Physical Behavior of Human Hair
Springer-Verlag New York - Berlin - Heidelberg - London - Paris - Tokyo
second Edition, New York 1988 (69 - 101)
Charles Zviak
The Science of Hair Care
Marcel Dekker Inc.
New York and Basel
New York, 1986 (183 - 212)
J.B. Wilkinson,
R.J. Moore
Harry's Cosmeticology
Verlag George Godwin London, Seventh Edition
London 1982 (555 - 586)
K.H. Schrader
Grundlagen und Rezepturen der Kosmetika
Hüthig Verlag 2. Auflage
Heidelberg 1989 (824 - 840)

## Tabelle 5.

Weitere brauchbare Verbindungen für die Anfertigung einer Spüllösung mit gegenüber den Fasern inerten Stoffen:

**Alanin** als Hydrochlorid, Acetat, Sulfat oder Phosphat
**Alanin** als Natrium, Kalium oder Ammoniumsalz

---

**Arginin** als Hydrochlorid, Acetat, Sulfat oder Phosphat
**Arginin** als Natrium-, Kalium- oder Ammoniumsalz

---

**Asparaginsäure** als Natrium-, Kalium- oder Ammoniumsalz

---

**Asparagin-Monohydrat** als Hydrochlorid, Acetat, Sulfat oder Phosphat
**Asparagin-Monohydrat** als Natrium-, Kalium- oder Ammoniumsalz

---

**Cytein** als Hxydrochlorid, Acetat, Sulfat oder Phosphat
**Cystein** als Natrium-, Kalium- oder Ammoniumsalz

---

**Glutaminsäure** als Hydrochlorid, Acetat, Sulfat oder Phosphat
**Glutaminsäure** als Natrium-, Kalium- oder Ammoniumsalz

---

**Histidin** als Hydrochlorid, Acetat, Sulfat oder Phosphat
**Histidin** als Natrium-, Kalium- oder Ammoniumsalz

---

**Leucin** als Hydrochlorid, Acetat, Sulfat oder Phosphat
**Leucin** als Natrium-, Kalium- oder Ammoniumsalz

---

**Methionin** als Hydrochlorid, Acetat, Sulfat oder Phosphat
**Methionin** als Natrium-, Kalium- oder Ammoniumsalz

---

**Phenylalanin** als Hydrochlorid, Acetat, Sulfat oder Phosphat
**Phenylalanin** als Natrium-, Kalium- oder Ammoniumsalz

---

**Valin** als Hydrochlorid, Acetat, Sulfat oder Phosphat
**Valin** als Natrium-, Kalium- oder Ammoniumsalz

---

**Guanidiniumcarbonat,** -acetat, -chlorid, -sulfat und -phosphat

---

**Harnstoff**

---

die Natrium-, Kalium-, Ammoniumsalze der **Ascorbinsäure**

---

die Natrium-, Kalium-, Ammoniumsalze der **Gluconsäure**

---

die Natrium-, Kalium-, Ammoniumsalze der $\alpha$-**Liponsäure**

---

die Mono- und Di-Natrium-, Kalium-, Ammoniumsalze
der **Glutarsäure**

---

das Di-, Tri-, Tetra-, Penta-, Hexa-, Hepta- und
Octa**ethylenglycol**

---

das **Propylenglycol, Dipropylenglykol, Tripropylenglycol**

---

das **Di-, Tri-, Tetra- und Pentaglycerin**

---

Mischpolymerisate des Ethylenoxids und Propylenoxids bis
zu einem mittleren Molekulargewicht von etwa 800.

---

**Glucose, Fructose, Maltose, Arabinose**

---

Die in der Beschreibung, in den Beispielen und in der Tabelle 5 genannten inerten Stoffe oder weitere inerte Stoffe, die zum Spülen bzw. Benetzen der Keratinfasern gemäß der Erfindung verwendet werden sollen, um eine Quellung derselben zu verhindern oder mindestens sehr deutlich herabzusetzen, können auf ihre Wirksamkeit mittels eines "Faser-Quellungs-Analysators" geprüft werden.

Ein derartiges Prüfgerät ist in dem Buch "16th I.F.S.C.C. Congress, Preprints Podium Presentations Volume 1, Cosmetic Science in the 1990's and beyond, October 8-11, 1990, The New York Hilton, New York City, Verlag International Federation Societies of Cosmetic Chemists, 1990, Seiten 315 bis 324 in der Arbeit "The influence of the osmotic swelling behaviour on the quality of the permanent wave, J. Nöthen, V. Böllert, G. Blankenburg, and H. Höcker" von Mitgliedern der Erfindergruppe beschrieben worden.

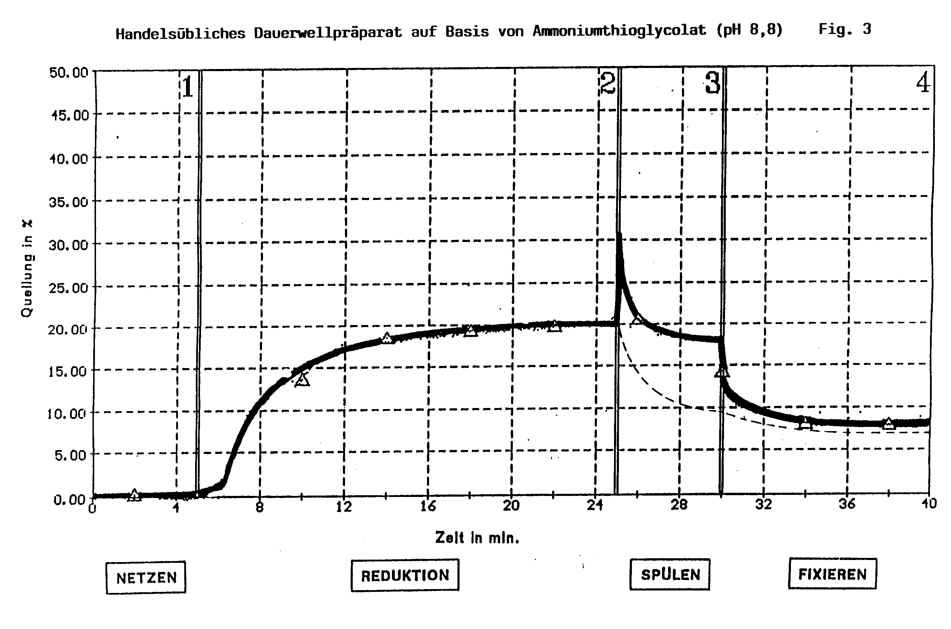

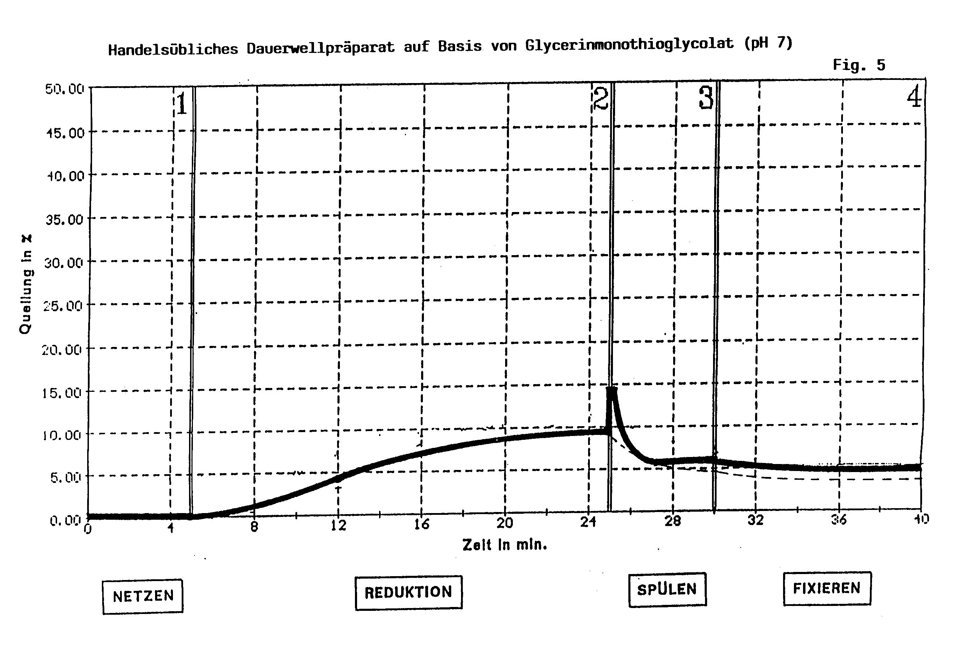

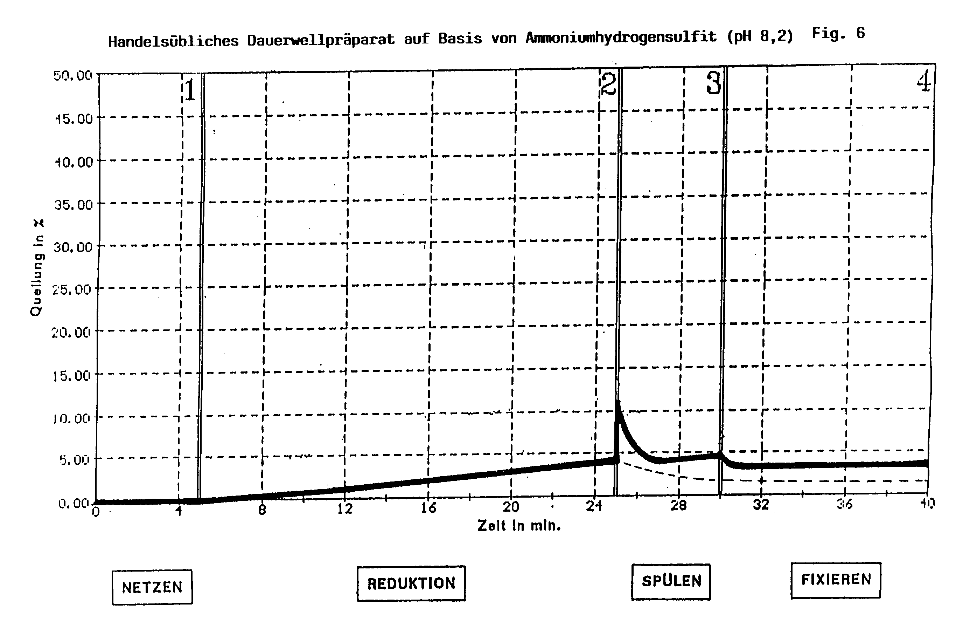

In Figur 3 bis Figur 6 sind die Kurven des Quellungsverlaufes von Keratinfasern während der Anwendung verschiedener handelsüblicher Klassen von Dauerwellpräparaten dargestellt. Bei den Figuren 3 bis 6 sind die verwendeten Dauerwellpräparate angegeben, nämlich Ammoniumthioglycolat (pH 8,8), Ammoniumthioglycolat (pH 6,8), Glycerinmonothioglycolat (pH 7,0) und Ammoniumhydrogensulfit (pH 8,2). Figur 3 stellt das Ergebnis gemäß dem Beispiel 3 dar.

Die durchgezogenen Linien entsprechen dem Quellungsverlauf der Keratinfasern bei herkömmlicher Spülung mit Wasser, die gestrichelten Linien zeigen die Ergebnisse und den Verlauf bei erfindungsgemäßer Spülung der Keratinfasern.

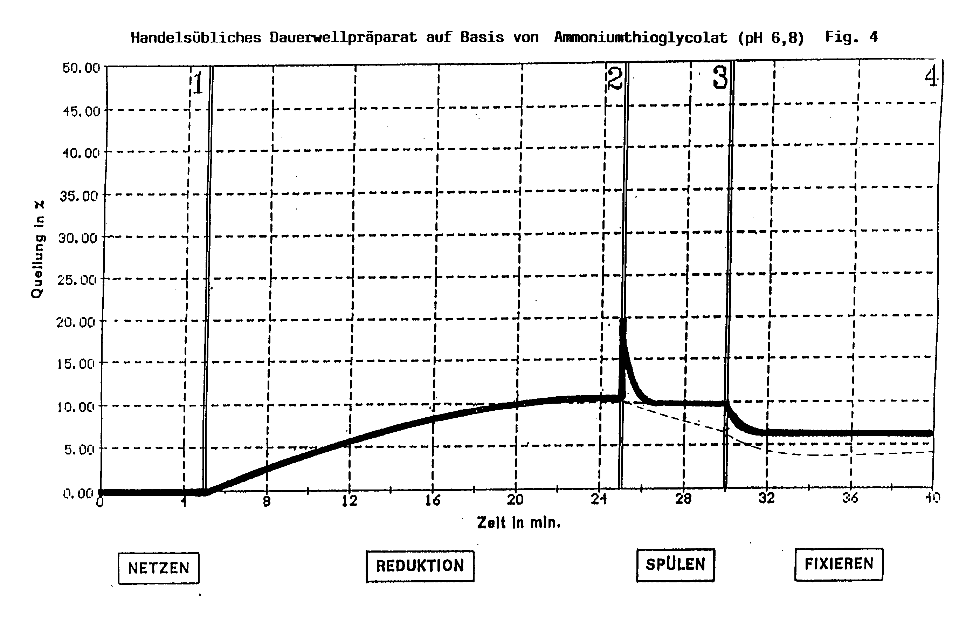

Bei den Untersuchungen, deren Ergebnisse in den Figuren 4, 5 und 6 wiedergegeben sind, wurde eine 10 gewichtsprozentige Kochsalzlösung zum Spülen verwendet.

**Patentansprüche**

1. Verfahren zur verformenden Behandlung von Keratinfasern, bei dem die Keratinfasern der Einwirkung einer reaktiven reduzierenden Lösung ausgesetzt werden, die Reduktionsmittel zum Aufbrechen der in den Fasern vorhandenen Cystin-Bindungen enthält, nach der Einwirkung dieser Lösung gespült wird, die Fasern der Einwirkung eines Oxidationsmittels ausgesetzt werden, um Cystin-Bindungen wieder zurückzubilden, und gegebenenfalls nochmals gespült wird, **dadurch gekennzeichnet,** daß nach dem Einwirken der reduzierenden Lösung mit einer solchen wässrigen Lösung von gegenüber den Fasern inerten Stoffen gespült wird, die bewirkt, daß zu keinem Zeitpunkt während des Spülvorganges der Durchmesser der Fasern um mehr als 30 % größer ist als am Ende des Reduktionsschrittes.

2. Verfahren nach Anspruch 1, dadurch gekennzeichnet, daß mit einer wässrigen Lösung gespült wird, die bewirkt, daß zu keinem Zeitpunkt während des Spülvorganges der Durchmesser der Fasern um mehr als 20 % größer ist als am Ende des Reduktionsschrittes.

3. Verfahren nach Anspruch 1, dadurch gekennzeichnet, daß mit einer wässrigen Lösung gespült wird, die bewirkt, daß zu keinem Zeitpunkt während des Spülvorganges der Durchmesser der Fasern um mehr als 10 % größer ist als am Ende des Reduktionsschrittes.

4. Verfahren nach Anspruch 1, dadurch gekennzeichnet, daß mit einer wässrigen Lösung gespült wird, die bewirkt, daß zu keinem Zeitpunkt des Spülvorganges der Durchmesser der Fasern zunimmt.

5. Verfahren zur verformenden Behandlung von Keratinfasern, bei dem die Keratinfasern der Einwirkung einer reaktiven reduzierenden Lösung ausgesetzt werden, die Reduktionsmittel zum Aufbrechen der in den Fasern vorhandenen Cystin-Bindungen enthält, nach der Einwirkung dieser Lösung gespült wird, die Fasern der Einwirkung eines Oxidationsmittels ausgesetzt werden, um Cystin-Bindungen wieder zurückzubilden, und gegebenenfalls nochmals gespült wird, dadurch gekennzeichnet, daß nach dem Einwirken der reduzierenden Lösung mit einer solchen wässrigen Lösung von gegenüber den Fasern inerten Stoffen gespült wird, die bewirkt, daß gegenüber dem Spülen mit destilliertem Wasser eine Verminderung der maximalen Zunahme des Faserdurchmessers von mindestens 20 % bewirkt wird.

6. Verfahren nach Anspruch 5, dadurch gekennzeichnet, daß mit einer wässrigen Lösung gespült wird, die bewirkt, daß gegenüber dem Spülen mit destilliertem Wasser eine Verminderung der maximalen Zunahme des Faserdurchmessers von mindestens 50 % bewirkt wird.

7. Verfahren nach einem der Ansprüche 1 bis 6, dadurch gekennzeichnet, daß die Spüllösung bei 25 °C gegenüber destilliertem Wasser einen osmotischen Druck von $6 \times 10^5$ bis $380 \times 10^5$ Pa aufweist.

8. Verfahren nach einem der Ansprüche 1 bis 7, dadurch gekennzeichnet, daß die Spüllösung eine wässrige Natriumchloridlösung ist.

9. Verfahren nach Anspruch 8, dadurch gekennzeichnet, daß die Natriumchloridlösung 10 g bis 350 g Natriumchlorid pro Liter Wasser enthält.

10. Variante des Verfahrens nach einem der Ansprüche 1 bis 9, dadurch gekennzeichnet, daß nach Beginn des Spülens mit der wässrigen Lösung, die die gegenüber den Fasern inerten Stoffe enthält, durch kontinuierliche oder diskontinuierliche Zugabe von Wasser die Konzentration der inerten Stoffe in der Spüllösung so lange abgesenkt wird, bis nur noch mit Wasser gespült wird.

11. Variante des Verfahrens nach einem der Ansprüche 1 bis 9, dadurch gekennzeichnet, daß

(1) nach dem Einwirken der reduzierenden Lösung in einer ersten Behandlungsstufe

(2) die Keratinfasern in einer zweiten Behandlungsstufe mit einer solchen wässrigen Lösung von gegenüber den Keratinfasern inerten Stoffen benetzt werden, die bewirkt, daß zu keinem Zeitpunkt während des Benetzungsvorganges der Durchmesser der Keratinfasern um mehr als 30% größer ist als am Ende des Reduktionsschrittes und im günstigsten Falle zu keinem Zeitpunkt des Benetzungsvorganges der Durchmesser der Keratinfasern zunimmt und

(3) in einer dritten Behandlungsstufe die benetzten Keratinfasern mit Wasser gespült werden, wobei zu keinem Zeitpunkt des Spülvorganges der Durchmesser der Keratinfasern zunimmt.

12. Verwendung eines Mittels zur Durchführung des Verfahrens nach einem der Ansprüche 1-11, das bei 25°C gegenüber destilliertem $H_2O$ einen osmotischen Druck von $6 \times 10^5$ bis $380 \times 10^5$ Pa aufweist.

13. Verwendung eines Mittels zur Durchführung des Verfahrens nach einem der Ansprüche 1 bis 11, wobei dieses 10 bis 350 g Natriumchlorid pro Liter Wasser enthält.

14. Verwendung eines Mittels zur Durchführung des Verfahrens nach einem der Ansprüche 1 bis 11, wobei dieses als gegen- Verbindungen, ausgewählt aus der Gruppe bestehend aus physiologisch verträglichen Natrium-, Ammonium-, Kalium-, Lithium-, Magnesium-, Aluminiumsalzen, Betain, Ethanol, Glycerin, D-Sorbit einzeln oder im Gemisch, enthält.

15. Verwendung eines Mittels zur Durchführung des Verfahrens nach Anspruch 14, wobei die Salze als Chloride, Bromide, Sulfate, Acetate, Citrate, Lactate einzeln oder im Gemisch enthalten sind.

16. Verwendung eines Mittels zur Durchführung des Verfahrens nach einem der Ansprüche 1 bis 11, wobei dieses Verbindungen nach den Ansprüchen 13, 14 und 15 als Gemisch enthält.


## Claims

1. Method for shaping treatment of keratin fibres, in which method the keratin fibres are subjected to the action of a reactive reducing solution, which contains reducing agents for breaking down the cystine bonds present in the fibres, and are rinsed after the action of this solution, and the fibres are subjected to the action of an oxidising agent, in order to reform cystine bonds, and, if appropriate, are rinsed again, characterised in that after the action of the reducing solution rinsing is carried out using an aqueous solution of substances which are inert with respect to the fibres, which solution has the effect that the diameter of the fibres is at no time during the rinsing process more than 30% larger than at the end of the reduction step.

2. Method according to Claim 1, characterised in that rinsing is carried out using an aqueous solution which has the effect that the diameter of the fibres is at no time during the rinsing operation more than 20% larger than at the end of the reduction step.

3. Method according to Claim 1, characterised in that rinsing is carried out using an aqueous solution which has the effect that the diameter of the fibres is at no time during the rinsing operation more than 10% larger than at the end of the reduction step.

4. Method according to Claim 1, characterised in that rinsing is carried out using an aqueous solution which has the effect that the diameter of the fibres does not increase at any time during the rinsing operation.

5. Method for shaping treatment of keratin fibres, in which method the keratin fibres are subjected to the action of a reactive reducing solution, which contains reducing agents to break down the cystine bonds present in the fibres, and are rinsed after the action of this solution, and the fibres are subjected to the action of an oxidising agent, in order to reform cystine bonds, and, if appropriate, rinsed again characterised in that after the action of the reducing solution, rinsing is carried out using an aqueous solution of substances which are inert with respect to the fibres, which solution has the effect that, compared with rinsing with distilled water, a reduction of at least 20% in the maximum increase in the fibre diameter is obtained.

6. Method according to Claim 5, characterised in that rinsing is carried out using an aqueous solution which has the effect that, compared with rinsing with distilled water, a reduction of at least 50% in the maximum increase in the fibre diameter is obtained.

7. Method according to one of Claims 1 to 6, characterised in that the rinse solution has an osmotic pressure of $6 \times 10^5$ to $380 \times 10^5$ Pa with respect to distilled water at 25°C.

8. Method according to one of Claims 1 to 7, characterised in that the rinse solution is an aqueous sodium chloride solution.

9. Method according to Claim 8, characterised in that the sodium chloride solution contains 10 g to 350 g of sodium chloride per litre of water.

10. Variant of the method according to one of Claims 1 to 9, characterised in that after the start of rinsing with the aqueous solution which contains substances inert with respect to the fibres, the concentration of the inert substances in the rinse solution is reduced by continuous or discontinuous addition of water until rinsing is carried out with water only.

11. Variant of the method according to one of Claims 1 to 9, characterised in that
    (1) after the action of the reducing solution in a first treatment step,
    (2) the keratin fibres are wetted in a second treatment step using an aqueous solution of substances which are inert with respect to the keratin fibres, which solution has the effect that at no time during the wetting operation is the diameter of the keratin fibres more than 30% larger than at the end of the reduction step and in the most advantageous case the diameter of the keratin fibres does not increase at any time during the wetting operation, and
    (3) in a third treatment step, the wetted keratin fibres are rinsed with water, the diameter of the keratin fibres not increasing at any time during the rinsing operation.

12. Use of an agent for carrying out the method according to one of Claims 1 to 11, which agent has an osmotic pressure of $6 \times 10^5$ to $380 \times 10^5$ Pa with respect to distilled $H_2O$ at 25°C.

13. Use of an agent for carrying out the method according to one of Claims 1 to 11, said agent containing 10 to 350 g of sodium chloride per litre of water.

14. Use of an agent for carrying out the method according to one of Claims 1 to 11, said agent containing compounds selected from the group comprising physiologically acceptable sodium, ammonium, potassium, lithium, magnesium or aluminium salts, betaine, ethanol, glycerol or D-sorbitol, on their own or as a mixture.

15. Use of an agent for carrying out the method according to Claim 14, the salts being present in the form of chlorides, bromides, sulphates, acetates, citrates or lactates, on their own or as a mixture.

16. Use of an agent for carrying out the method according to one of Claims 1 to 11, said agent containing compounds according to Claims 13, 14 and 15 as a mixture.

## Revendications

1. Procédé de traitement de déformation de fibres de kératine, dans lequel les fibres de kératine sont exposées à l'action d'une solution réactive réductrice qui contient des agents de réduction pour la rupture des liaisons cystine présentes dans les fibres, on rince après l'action de cette solution, on expose les fibres à l'action d'un agent d'oxydation de manière à reformer à nouveau les liaisons cystine, et le cas échéant on rince encore, procédé caractérisé en ce qu'après l'action de la solution réductrice on rince avec une solution aqueuse de substances inertes vis-à-vis des fibres, qui fait qu'à aucun moment pendant le processus de rinçage le diamètre des fibres est plus grand de plus de 30 % qu'à la fin de l'étape de réduction.

2. Procédé selon la revendication 1, caractérisé en ce que l'on rince avec une solution aqueuse qui occasionne, qu'à aucun moment pendant le processus de rinçage le diamètre des fibres est plus grand de plus de 20 % qu'à la fin de l'étape de réduction.

3. Procédé selon la revendication 1, caractérisé en ce que l'on rince avec une solution aqueuse qui occasionne, qu'à aucun moment pendant le processus de rinçage le diamètre des fibres est plus grand de plus de 10 % qu'à la fin de l'étape de réduction.

4. Procédé selon la revendication 1, caractérisé en ce que l'on rince avec une solution aqueuse, qui occasionne, qu'a aucun moment du processus de rinçage, le diamètre des fibres augmente.

5. Procédé pour le traitement déformant des fibres de kératine, dans lequel les fibres de kératine sont exposées à l'action d'une solution réactive réductrice qui contient des agents réducteurs pour la rupture des liaisons cystine présentes dans les fibres, on rince après l'action de cette solution, on expose les fibres à l'action d'un agent d'oxydation de façon à reformer à nouveau des liaisons cystine et le cas échéant on rince encore une fois, procédé caractérisé en ce qu'après l'action de la solution réductrice on rince avec une solution aqueuse de substances inertes vis-à-vis des fibres, qui fait, que par rapport au rinçage avec de l'eau distillée, on cause une diminution de l'augmentation maximale du diamètre des fibres d'au moins 20 %.

6. Procédé selon la revendication 5, caractérisé en ce qu'on rince avec une solution aqueuse qui provoque, que par rapport au rinçage avec de l'eau distillée, on cause une diminution de l'augmentation maximale du diamètre des fibres d'au moins 50 %.

7. Procédé selon l'une des revendications 1 à 6, caractérisé en ce que la solution de rinçage possède à 25°C vis-à-vis de l'eau distillée une pression osmotique de $6 \times 10^5$ à $380 \times 10^5$ Pa.

8. Procédé selon l'une des revendications 1 à 7, caractérisé en ce que la solution de rinçage est une solution aqueuse de chlorure de sodium.

9. Procédé selon la revendication 8, caractérisé en ce que la solution de chlorure de sodium renferme de 10 g à 350 g de chlorure de sodium par litre d'eau.

10. Variante du procédé selon l'une des revendications 1 à 9, caractérisée en ce qu'après le commencement du rinçage avec la solution aqueuse, qui contient des substances inertes vis-à-vis des fibres, la concentration en substances inertes se trouve abaissée par addition continue ou discontinue d'eau, dans la solution de rinçage aussi longtemps jusqu'à ce que l'on ne rince plus qu'avec de l'eau.

11. Variante du procédé selon l'une des revendications 1 à 9, caractérisée en ce que
(1) après l'action de la solution réductrice dans une première étape de traitement,
(2) les fibres de kératine dans une deuxième étape de traitement sont humectées avec une solution aqueuse de substances inertes vis-à-vis des fibres de kératine pour qu'à aucun moment pendant le processus d'humidification, le diamètre des fibres kératiniques est plus grand de plus de 30 % qu'à la fin de l'étape de réduction et dans le cas le plus favorable le diamètre des fibres kératiniques à aucun moment du processus d'humidification n' augmente et
(3) dans une troisième étape de traitement les fibres de kératine humectées sont rincées à l'eau, étape dans laquelle à aucun moment du processus de rinçage le diamètre des fibres de kératine n'augmente.

12. Utilisation d'un agent pour l'exécution du procédé selon l'une des revendications 1 à 11, qui possède à 25°C - vis-à-vis de l'eau distillée - une pression osmotique de $6 \times 10^5$ à $380 \times 10^5$ Pa.

13. Utilisation d'un agent pour l'exécution du procédé selon l'une des revendications 1 à 11, cet agent contenant de 10 à 350 g de chlorure de sodium par litre d'eau.

14. Utilisation d'un agent pour l'exécution du procédé selon l'une des revendications 1 à 11 cet agent contenant des composés choisis dans le groupe formé de sels de sodium, d'ammonium, de potassium, de lithium, de magnésium, d'aluminium physiologiquement compatibles, de la bétaïne, de l'éthanol, du glycérol, du D-sorbitol, isolément ou en mélange.

15. Utilisation d'un agent pour l'exécution du procédé selon la revendication 14, agent dans lequel les sels sous forme de chlorures, bromures, sulfates, acitates, citrates et lactates sont contenus isolément ou en mélange.

16. Utilisation d'un agent pour l'exécution du procédé selon l'une des revendications 1 à 11 dans lequel celui-ci renfermant des composés selon les revendications 13, 14 et 15 sous forme de mélange.

Fig. 1

Fig. 2

Handelsübliches Dauerwellpräparat auf Basis von Ammoniumthioglycolat (pH 8,8)   Fig. 3

Handelsübliches Dauerwellpräparat auf Basis von Ammoniumthioglycolat (pH 6,8)  Fig. 4

Handelsübliches Dauerwellpräparat auf Basis von Glycerinmonothioglycolat (pH 7)

Fig. 5

Quellung in %

NETZEN

REDUKTION

SPÜLEN

FIXIEREN

Zeit in min.

Handelsübliches Dauerwellpräparat auf Basis von Ammoniumhydrogensulfit (pH 8,2)   Fig. 6

NETZEN    REDUKTION    SPÜLEN    FIXIEREN